(19) Europäisches Patentamt
European Patent Office
Office européen des brevets

(11) Numéro de publication: **0 260 185 B1**

(12) # FASCICULE DE BREVET EUROPEEN

(45) Date de publication du fascicule du brevet: **28.11.90**

(51) Int. Cl.[5]: **C07C 319/06**

(21) Numéro de dépôt: **87401969.8**

(22) Date de dépôt: **02.09.87**

(54) **Procédé catalytique de production de mercaptans à partir de thioéthers.**

(30) Priorité: **11.09.86 FR 8612690**

(43) Date de publication de la demande: **16.03.88 Bulletin 88/11**

(45) Mention de la délivrance du brevet: **28.11.90 Bulletin 90/48**

(84) Etats contractants désignés: **BE DE ES FR GB IT NL**

(56) Documents cités:
**EP-A- 0 047 021**
**US-A- 4 005 149**
**US-A- 4 059 636**

(73) Titulaire: **SOCIETE NATIONALE ELF AQUITAINE (PRODUCTION), Tour Elf, 2, Place de la Coupole, La Défense 6, F-92400 Courbevoie(FR)**

(72) Inventeur: **Arretz, Emmanuel Felix Etienne, 2, rue de Cagnes, F-64000 Pau(FR)**

(74) Mandataire: **Leboulenger, Jean et al, ATOCHEM Département Propriété Industrielle, F-92091 Paris la Défense 10 Cédex 42(FR)**

**EP 0 260 185 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention se rapporte à la production de mercaptans à partir de thioéthers et de l'hydrogène sulfuré, en présence de catalyseurs appropriés.

L'intérêt industriel des mercaptans fait que de nombreux travaux ont été effectués en vue de la mise au point de la fabrication de ces composés. Les procédés, les plus employés dans la pratique, sont basés sur la réaction de l'hydrogène sulfuré avec des alcools ou avec des oléfines. Dans la majorité des cas, les principaux sous-produits obtenus sont des thioéthers, principalement formés par réaction des mercaptans avec les alcools ou avec les oléfines, suivant le procédé utilisé. A part un nombre réduit de thioéthers, les thioéthers symétriques obtenus dans la fabrication des mercaptans n'ont généralement pas de valorisation commerciale.

Des méthodes de conversion de ces thioéthers ont été proposées pour les transformer en mercaptans correspondants par réaction avec l'hydrogène sulfuré en présence de différents catalyseurs.

Cependant, malgré certaines améliorations apportées (catalyseurs plus performants, introduction de sulfure de carbone comme promoteur...) ces réactions nécessitent des températures élevées (250 à 360°C) et conduisent à la formation de sous-produits indésirables qui ont pour conséquence de réduire sensiblement la sélectivité en mercaptan.

Dans le document EP-A 47 021 est décrit un procédé de préparation d'alkylmercaptans par réaction d'un dialkyl sulfure avec l'hydrogène sulfuré en présence d'une zéolithe comme catalyseur. La réaction est menée à une température élevée comprise entre 250 et 450°C, de préférence entre 320 et 390°C. Les dialkyl sulfures R-S-R mis en œuvre sont toujours des thioéthers symétriques, par exemple le diméthyl sulfure, le diéthyl sulfure, le di-tert.butyl sulfure, le dicyclohexyl sulfure, le di-n.octyl sulfure et le di-n.dodécyl sulfure.

La présente invention apporte un procédé nouveau, dont les principaux avantages sont un rendement et une sélectivité très élevés en mercaptan, et la possibilité d'obtention aisée de mercaptans primaires ou secondaires. Le procédé suivant l'invention consistant à faire réagir de l'hydrogène sulfuré avec un thioéther en présence d'un catalyseur acide approprié, est caractérisé en ce que l'on utilise un thioéther dont l'atome de soufre est lié d'une part à un carbone tertiaire et d'autre part à un carbone primaire ou secondaire (voir formule A ci-après). La réaction a lieu à une température comprise entre la température ambiante et 200°C, avantageusement au-dessus de 80°C, de préférence sous pression, le thioéther étant à l'état liquide ou gazeux.

La sulfhydrolyse du thioéther, suivant l'invention, peut être représentée par la réaction :

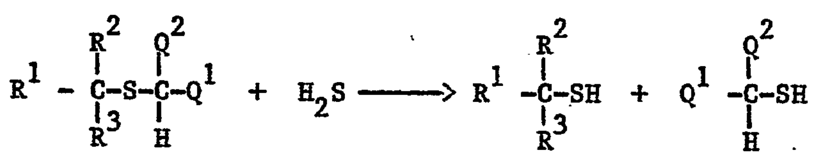 (1)

(A) (B) (C)

où $R^1$, $R^2$ et $R^3$, semblables ou différents, sont des radicaux hydrocarbonés, notamment alkyles, cycloalkyles, ou/et aryles, l'atome de carbone qui les porte étant ainsi tertiaire ; les symboles $Q^1$ et $Q^2$ ont la même signification que $R^1$, $R^2$ et $R^3$, mais l'un deux peut aussi être un atome d'hydrogène ou porter un ou plusieurs groupes fonctionnels.

On obtient donc, à partir du thioéther (A) un mercaptan tertiaire (B) et un mercaptan (C) qui peut être primaire ou secondaire, selon la nature des groupes Q.

Cette nouvelle réaction fournit de vastes possibilités de fabrication. Si l'on possède une source économique de thioéther (A) et une utilisation des deux mercaptans (B) et (C), on peut recueillir ces deux produits à la fois. Une variante très intéressante de fabrication du composé (C) seul consiste à réutiliser (B) pour produire à nouveau du thioéther (A) à partir d'une oléfine

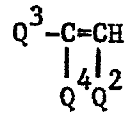

par la réaction :

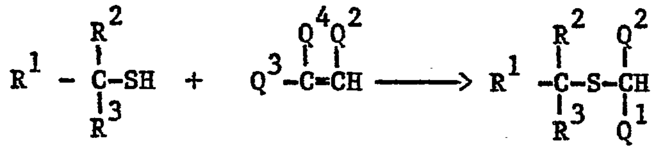

(2)

(B) (A)

$Q^1$ désignant

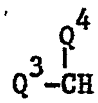

où $Q^3$ et $Q^4$ sont des hydrogènes ou des groupes hydrocarbonés.

Suivant le type de thioéther (A), sa synthèse est réalisée, soit par un processus radicalaire ou/et photochimique, soit par une réaction de catalyse hétérogène.

L'obtention de thioéther (A) peut être aussi envisagée par d'autres voies de synthèse connues, suivant l'intérêt technico-économique d'utiliser d'autres matières premières que des oléfines, à savoir des dérivés halogénés, des alcools, etc. Celui-ci peut ainsi être utilisé cycliquement, avec production avantageuse de mercaptan (C), au moyen des deux réactions qui viennent d'être décrites, et qui, dans la pratique, sont réalisées dans l'ordre successif (2) et (1).

Bien que le nouveau procédé puisse être réalisé avec des composés à groupes $R^1$, $R^2$, $R^3$ et $Q^1$, $Q^2$, $Q^3$, $Q^4$ très variés, les applications les plus courantes portent sur des $R^1$ à $R^3$ choisis parmi les alkyles en $C_1$ à $C_{24}$ et surtout en $C_1$ à $C_{18}$ ou/et des cycloalkyles et aryles en $C_5$ à $C_{10}$ pouvant porter des substitutions alkyliques. Il en est de même des $Q^1$ à $Q^4$, sauf que plusieurs d'entre eux peuvent être des atomes d'hydrogène ou porter des groupes fonctionnels, tels que CN, COOH, OH, $NH_2$, halogène, ester, étheroxyde, amide, S non tertiaire, sulfoxyde, sulfone etc..

Lorsque le procédé cyclique, susmentionné, est utilisé, on a intérêt à choisir les $R^1$ à $R^3$ tels que le mercaptan (B), c'est-à-dire $R^1$ $R^2$ $R^3$ -C-SH présente une température d'ébullition suffisamment différente de celle du mercaptan (C) à préparer, $Q^1$ $Q^2$ -HC-SH, afin que leur séparation par distillation soit facile.

Ainsi, par exemple, l'invention permet-elle de réaliser presque quantitativement une réaction telle que :

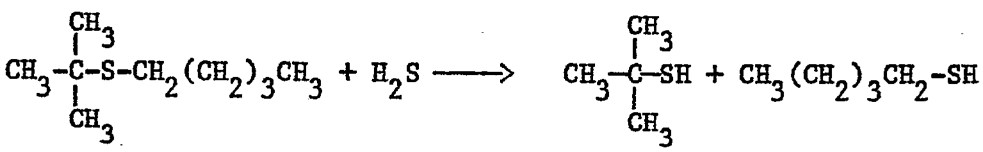

sulfure de tert.butyle et n-amyle (A') | tertiobutyl mercaptan Eb. 66°C | n-amyl mercaptan Eb. 126°C

(1')

Il est donc aisé de séparer par distillation ces deux composés, et utiliser le premier pour reconstituer le sulfure (thioéther) de départ, par une réaction photochimique ou radicalaire :

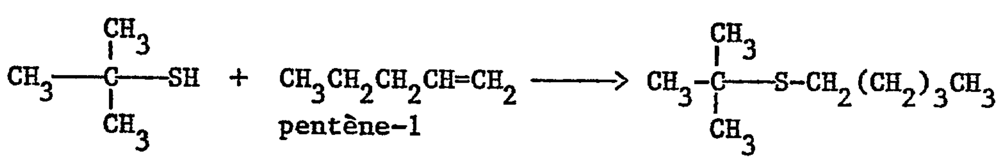

pentène-1

(2')

(A')

Le thioéther (A') sert à nouveau à la production de n-amyl mercaptan suivant la réaction (1'), et ainsi de suite.

Les catalyseurs préférés pour la présente invention sont des aluminosilicates naturels et synthétiques, comme les zéolithes, ou encore des résines échangeuses d'ions fortement acides. Parmi les aluminosilicates naturels on peut citer les silices-alumines ayant des teneurs en alumine de 1 à 20 %, comme celles que produit la Société DAVISON CHEMICAL. Soit encore des dérivés de la montmorillonite acides, de structure générale :

$(Mg\text{-}Ca)O.Al_2O_3.5SiO_2nH_2O$

dont la marque commerciale est FILTROL.

En ce qui concerne les zéolithes, il s'agit de préférence des types X et Y à teneurs en métal alcalin inférieures à 15 % sous forme de $Na_2O$, et plus avantageusement à moins de 3 % en poids de métal alcalin.

Quant aux résines échangeuses d'ions, ce sont des échangeurs cationiques acides, à structure polymérique, aromatique, ou à structure aliphatique perfluorée, contenant des groupes sulfoniques, et pouvant être utilisées en masse ou fixées à un support minéral ou organique.

Ces matières polymériques peuvent être à base de polystyrène et divinylbenzène. Dans ce cas, il s'agit de résines sulfoniques, que l'on trouve dans le commerce sous l'appellation d'Amberlite, Amberlyst, Lewatit, Dowex, Duolite. Quant aux acides perfluorosulfoniques ils sont commercialisés par DU PONT DE NEMOURS sous le nom de NAFIONS.

L'acide phosphorique imprégnant un support peut aussi convenir comme catalyseur.

Comme indiqué plus haut, la sulfhydrolyse (1) suivant l'invention s'opère à des températures comprises entre la température ambiante et 200°C (de préférence au-dessus de 80°C) selon la nature du thioéther traité. Dans les cas les plus courants, où ce thioéther compte environ 6 à 24 atomes de carbone, la température optimale se situe entre environ 100°C et 180°C et surtout entre 110°C et 155°C.

Suivant les cas, la durée de contact du thioéther et de 1'$H_2S$ avec le catalyseur varie en général entre 15 et 120 mn.

La réaction peut être réalisée sous une légère pression seulement, mais, pour améliorer sensiblement la production et éviter des réactions secondaires, il est bon d'opérer sous des pressions plus fortes, notamment sous 5 à 30 bars, et surtout sous 10 à 20 bars.

Il est avantageux, dans la conduite du procédé suivant l'invention, d'opérer avec un certain excès d'$H_2S$ par rapport à la quantité stoechiométrique de thioéther. Ainsi est-il recommandable d'opérer avec 1 à 6 moles d'$H_2S$ par mole de thioéther mis en oeuvre, et surtout avec 2 à 4 moles $H_2S$.

Dans la mode le plus pratique d'exécution de l'invention, un mélange, homogène ou hétérogène, de 1'$H_2S$ avec le thioéther à l'état de vapeur ou liquide, passe à travers la charge de catalyseur placée dans un réacteur maintenu à la température et la pression voulues.

Les produits, obtenus après contact des réactifs sur ces catalyseurs, sont soumis généralement à une distillation fractionnée. Dans le procédé cyclique, le mercaptan tertiaire formé est recyclé au réacteur de production du thioéther, tandis que le thioéther non converti est renvoyé à l'entrée du réacteur de sulfhydrolyse pour être à nouveau traité. Des mercaptans primaires ou secondaires peuvent être, dans les conditions de la présente invention, obtenus avec une pureté supérieure à 99 %.

Les exemples suivants ne sont pas limitatifs, ils montrent les possibilités offertes par la présente invention et l'intérêt pratique du nouveau procédé.

**EXEMPLE 1 :** Préparation simultanée de tertio-butyl mercaptan et de n-dodécylmercaptan

On se sert d'un réacteur tubulaire de 25 mm de diamètre, présentant une capacité utile de 200 ml chargée de 200 ml de résine échangeuse de cations, connue sous la dénomination commerciale d'AMBERLYST 15, préalablement séchée. A travers cette charge on fait passer à l'heure : 84 g de thioéther constitué par du sulfure de tertiobutyle et de n-dodécyle, $(CH_3)_3C\text{-}S\text{-}CH_2(CH_2)_{10}CH_3$, et 45 g de $H_2S$ (soit 4 moles $H_2S$ pour 1 de sulfure).

La pression, dans le réacteur, est maintenue à 15 bars, la température est réglée à 110° ± 2°C par la circulation d'huile, maintenue à température constante, à travers une double enveloppe qui entoure le réacteur.

L'analyse des produits bruts de la réaction montre que la transformation du thioéther de départ est de 65 % donne uniquement due tertio-butyl mercaptan $(CH_3)_3C\text{-}SH$ et du n-dodécyl mercaptan $CH_3(CH_3)_{10}CH_2\text{-}SH$ ; la production de ce dernier est de 43 g/heure avec une sélectivité égale pratiquement à 100 %.

**EXEMPLE 2 :** Réutilisation cyclique du mercaptan tertiaire

Les produits, obtenus à la suite de l'opération de l'exemple 1, ont été séparés les uns des autres par distillation, ce qui était facilité par les écarts substantiels entre leurs points d'ébullition.

Après récupération du n-dodécyl mercaptan en tant que produit voulu de la fabrication, on a fait passer le tertiobutyl mercaptan dans un réacteur photochimique de la façon suivante.

Le réacteur est constitué par un cylindre en acier inoxydable, d'un volume utile de 300 ml ; dans l'axe de ce cylindre est fixé un tube coaxial en quartz, contenant une lampe à mercure dont le maximum d'émission se situe à la longeur d'onde de 350 nm.

Le refroidissement et l'agitation du milieu réactionnel sont assurés par une boucle extérieure, comprenant une pompe de recirculation et un échangeur permettant de maintenir la température du milieu à 20°C ± 2°C.

Dans le réacteur on introduit en continu, par heure, 135 g de tertiobutyl mercaptan $(CH_3)_3C\text{-}SH$, soit 1,5 mole, et 168 g de dodécène-1, c'est-à-dire 1 mole, additionnés de 2,2 x $10^{-3}$ mole de benzophénone et 0,6 x $10^{-3}$ mole de phosphite tributylique. Le débit du liquide sortant du réacteur est mesuré ; par distillation on en élimine l'excès de mercaptan et le dodécène non transformé ; le poids de thioéther formé par la réaction

$$(CH_3)_3C\text{-}SH + CH_2\text{=}CH\text{-}(CH_2)_9\text{-}CH_3 \longrightarrow (CH_3)_3C\text{-}S\text{-}CH_2\text{-}(CH_2)_{10}CH_3$$

est de 129 g par heure, correspondant à un rendement de 50 % par rapport au dodécène mis en oeuvre. Ce thioéther est utilisé dans le procédé de l'exemple 1 pour la production de n-dodécyl mercaptan et ce cycle opératoire est répété 10 fois : on obtient ainsi du n-dodécyl mercaptan avec un taux de transformation moyen du dodécène de 77,9 % et avec une sélectivité de 99 % sur cette oléfine.

**EXEMPLE 3 :** Emploi d'un silico-aluminate comme catalyseur

Les opérations de l'exemple 1 ont été répétées avec un dérivé de la montmorillonite acide de structure $(Mg\text{-}Ca)O.Al_2O_3.\text{-}5SiO_2.nH_2O$, connu dans l'industrie sous la marque de FILTROL. 200 ml de ce catalyseur étaient placés dans le réacteur au lieu de l'AMBERLYST.

Le taux de transformation du thioéther était de 59 % et les mercaptans obtenus étaient aussi purs que dans l'exemple 1.

**EXEMPLE 4 :** Sulfhydrolyse du sulfure de tertiooctyle et n-butyle

Le thioéther correspondant à cet exemple a été synthétisé par addition radicalaire du tertiooctylmercaptan, produit d'origine commerciale, sur du butène-1. Le but de la réaction de sulfhydrolyse est de transformer ce thioéther en deux mercaptans : le tertiooctylmercaptan et le n-butylmercaptan suivant la réaction :

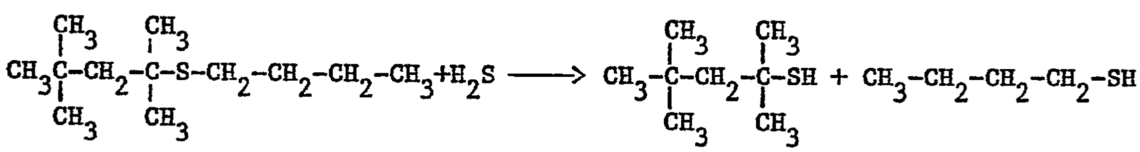

Les conditions opératoires de l'exemple 1, c'est-à-dire 110°C, 15 bars et 200 ml de résine AMBERLYST comme catalyseur, sont appliquées à la réaction.

Le débit horaire du thioéther est de 34 g et celui de 1'$H_2S$ 45 g (soit $4H_2S$ par mole de thioéther).

On obtient ainsi le n-butyl mercaptan $CH_3CH_2CH_2CH_2SH$ avec une sélectivité pratiquement de 100 %, à raison de 20 g/h, 66 % de thioéther étant transformés.

Comme ce mercaptan bout à 98°C contre 150°C pour le tertiooctyl mercaptan, sa séparation est aisée. Ce dernier est réutilisé pour la production d'une nouvelle charge de sulfure de tert.olctyle et n-butyle en vue d'une nouvelle réaction susindiquée.

**EXEMPLE 5 :** Sylfhydrolyse du sulfure de tertiobutyle et d'isopropyle

Le thioéther de départ a été obtenu par une réaction entre le tertiobutylmercaptan et le chlorure d'isopropyle catalysée par la soude.

La réaction de sulfhydrolyse a pour but de transformer le thioéther en isopropylmercaptan et en tertiobutylmercaptan suivant le schéma :

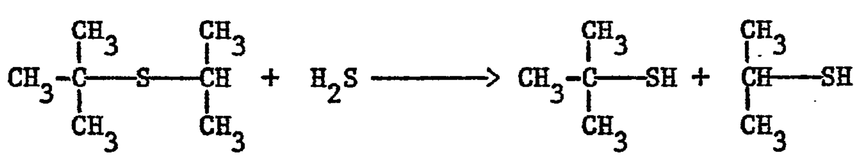

Les conditions de l'exemple 1 ont été reprises, à savoir 110°C, 15 bars et 200 ml de résine Amberlyst comme catalyseur. Le débit horaire de thioéther est de 43,5 g et celui de $H_2S$ 45 g (soit $4H_2S$ par mole de thioéther).

On obtient l'isopropylmercaptan avec une sélectivité pratiquement de 100 %, à raison de 17.8 g/h, 71 % de thioéther étant transformés.

**EXEMPLE 6 :** Sulfhydrolyse de l'acide ω-tertiobutylmercaptoundécanoïque

Le réactif sulfure-acide a été préalablement préparé par réaction catalysée par la soude entre le tertiobutylmercaptan et l'acide bromo-11 undécanoïque. La réaction de sulfhydrolyse permet d'obtenir l'acide mercapto-11 undécanoïque et de récupérer le tertiobutylmercaptan.

Cette réaction peut être schématisée ainsi :

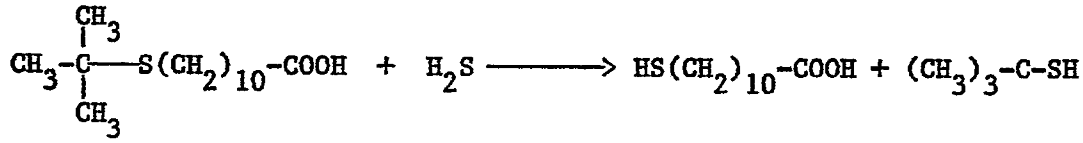

Les conditions de l'exemple 1 ont été reprises, à savoir 110°C, 15 bars et 200 ml de résine Amberlyst 15 comme catalyseur. Le sulfure-acide est introduit à raison de 0,32 mole/h en solution dans l'heptane tandis que l'hydrogène sulfuré est injecté gazeux dans le réacteur sur la base de 1,30 mole/h.

On obtient une production de 41,8 g/h d'acide mercapto-11 undécanoïque avec une sélectivité pratiquement de 100 %.

La transformation du sulfure-acide de départ est de 60 %.

## Revendications

1. Procédé catalytique pour la production de mercaptans à partir de thioéthers, dans lequel un thioéther est mis à réagir avec de l'hydrogène sulfuré en présence d'un catalyseur acide approprié, caractérisé en ce que le thioéther utilisé est de type

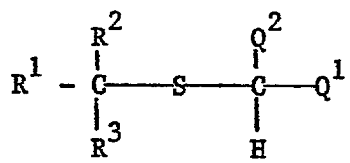

où les groupes $R^1$, $R^2$ et $R^3$, ainsi que $Q^1$ et $Q^2$, semblables ou différents, sont des alkyles, cycloalkyles ou/et aryles, l'un des symboles $Q^1$ et $Q^2$ pouvant désigner un atome d'hydrogène ou porter des fonctions.

2. Procédé suivant la revendication 1, caractérisé en ce que les groupes $R^1$ à $R^3$ et $Q^1$ et $Q^2$, lorsqu'ils sont des alkyles, sont en $C_1$ à $C_{24}$ et particulièrement en $C_1$ à $C_{18}$, et en $C_5$ à $C_{10}$ quand ils sont des cycloalkyles ou des aryles.

3. Procédé suivant la revendication 1 ou 2, caractérisé en ce que le catalyseur acide est un échangeur d'ions, en particulier résine ou zéolithe.

4. Procédé suivant la revendication 1 ou 2, caractérisé en ce que le catalyseur acide est un silico-aluminate, en particulier du type montmorillonite.

5. Procédé suivant la revendication 1 ou 2, caractérisé en ce que le catalyseur acide est une résine échangeuse d'ions à structure polymérique, aromatique, soit encore à structure aliphatique perfluorée contenant des groupes sulfoniques et pouvant être utilisée en masse ou fixée à un support minéral ou organique.

6. Procédé suivant la revendication 1 ou 2, caractérisé en ce que le catalyseur acide est l'acide phosphorique imprégnant un support.

7. Procédé suivant une des revendications 1 à 6, caractérisé en ce qu'il est réalisé à température comprise entre la température ambiante et 200°C.

8. Procédé suivant une des revendications 1 à 7, caractérisé en ce que l'on opère sous une pression de 5 à 30 bars, plus spécialement sous 10 à 20 bars.

9. Procédé suivant une des revendications 1 à 8, caractérisé en ce que la proportion de $H_2S$ utilisé est de 1 à 6 moles, et en particulier 2 à 4 moles, per mole de thioéther.

10. Procédé suivant une des revendications 1 à 9, caractérisé en ce que le mercaptan tertiaire, ($R^1$ $R^2$ $R^3$)-C-SH, formé est séparé du mercaptan primaire ou secondaire $Q^1$ $Q^2$ CH-SH, et utilisé pour la production d'une nouvelle quantité de thioéther, par réaction avec une oléfine ou tout autre réactif susceptible de donner le thioéther, et le thioéther sert à nouveau à une préparation selon la revendication 1.

11. Procédé cyclique suivant la revendication 10, caractérisé en ce qu'au moins un des groupes $Q^1$ et $Q^2$ porte un groupe fonctionnel, notamment -COOH, -CN, -OH, $-NH_2$, halogène, étheroxyde, ester, amide, sulfure non tertiaire, sulfoxyde ou sulfone.

12. Procédé suivant la revendication 10, caractérisé en ce que

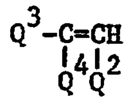 dans laquelle 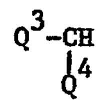

précédemment décrit et/ou $Q^3$ et $Q^4$ sont des hydrogènes ou des groupes hydrocarbonés.

## Patentansprüche

1. Katalytisches Verfahren zur Herstellung von Mercaptanen ausgehend von Thioethern, in dem ein Thioether mit Schwefelwasserstoff in Gegenwart eines geeigneten sauren Katalysators umgesetzt wird, dadurch gekennzeichnet, daß der eingesetzte Thioether vom Typ

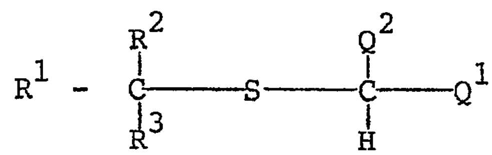

ist, worin die Gruppen $R^1$, $R^2$ und $R^3$ sowie $Q^1$ und $Q^2$ gleich oder verschieden und Alkyle, Cycloalkyle und/oder Aryle sind und eines der Symbole $Q^1$ und $Q^2$ ein Wasserstoffatom bezeichnen oder funktionelle Gruppen besitzen kann.

2. Verfahren nach Anspruch 1, dadurch gekennzeichnet, daß die Gruppen $R^1$ bis $R^3$ und $Q^1$ und $Q^2$, wenn sie Alkyle sind, $C_1$ bis $C_{24}$- und insbesondere $C_1$ bis $C_{18}$-Alkyle sind und wenn sie Cycloalkyle oder Aryle $C_5$- bis $C_{10}$-Cycloalkyle oder Aryle sind.

3. Verfahren nach Anspruch 1 oder 2, dadurch gekennzeichnet, daß der saure Katalysator ein Ionenaustauscher, insbesondere Harz oder Zeolith ist.

4. Verfahren nach Anspruch 1 oder 2, dadurch gekennzeichnet, daß der saure Katalysator ein Silicoaluminat, insbesondere vom Montmorillonit-Typ ist.

5. Verfahren nach Anspruch 1 oder 2, dadurch gekennzeichnet, daß der saure Katalysator ein Ionenaustauscherharz mit polymerer, aromatischer oder mit perfluorierter aliphatischer Struktur ist, das Sulfongruppen enthält und als Masse oder fixiert auf einen mineralischen oder organischen Träger eingesetzt werden kann.

6. Verfahren nach Anspruch 1 oder 2, dadurch gekennzeichnet, daß der saure Katalysator eine einen Träger durchtränkende Phosphorsäure ist.

7. Verfahren nach einem der Ansprüche 1 bis 6, dadurch gekennzeichnet, daß es bei einer Temperatur zwischen Umgebungstemperatur und 200°C durchgeführt wird.

8. Verfahren nach einem der Ansprüche 1 bis 7, dadurch gekennzeichnet, daß man bei einem Druck von 5 bis 30 bar, insbesondere bei 10 bis 20 bar, arbeitet.

9. Verfahren nach einem der Ansprüche 1 bis 8, dadurch gekennzeichnet, daß der eingesetzte $H_2S$-Anteil 1 bis 6 mol und insbesondere 2 bis 4 mol pro mol Thioether beträgt.

10. Verfahren nach einem der Ansprüche 1 bis 9, dadurch gekennzeichnet, daß das gebildete tertiäre Mercaptan ($R^1$ $R^2$ $R^3$)–C–SH vom primären oder sekundären $Q^1$ $Q^2$ CH–SH abgetrennt wird und zur Herstellung einer neuen Thioethermenge durch Reaktion mit einem Olefin oder irgendeinem anderen zur Bildung von Thioether geeigneten Mittel eingesetzt wird und der Thioether von neuem zur Herstellung nach Anspruch 1 dient.

11. Cyclisches Verfahren nach Anspruch 10, dadurch gekennzeichnet, daß mindestens eine der Gruppen $Q^1$ und $Q^2$ eine funktionelle Gruppe, insbesondere –COOH, –CN, –OH, $-NH_2$, Halogen, Etheroxid, Ester, Amid, nicht tertiäres Sulfid, Sulfoxid oder Sulfon besitzt.

12. Verfahren nach Anspruch 10, dadurch gekennzeichnet, daß das Olefin von der Struktur

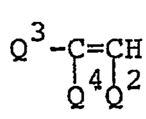

ist, in der

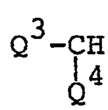

das zuvor beschriebene $Q^1$ bedeutet und/oder $Q^3$ und $Q^4$ Wasserstoff- oder Kohlenwasserstoffgruppen sind.

## Claims

1. Catalytic process for the production of mercaptans from thioethers, in which a thioether is reacted with hydrogen sulphide in the presence of a suitable acidic catalyst, characterized in that the thioether employed is of the the type

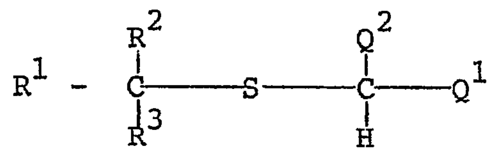

where the groups $R^1$, $R^2$ and $R^3$, as well as $Q^1$ and $Q^2$, which are similar or different, are alkyls, cycloalkyls and/or aryls, it being possible for one of the symbols $Q^1$ and $Q^2$ to denote a hydrogen atom or to carry functional groups.

2. Process according to Claim 1, characterized in that the groups $R^1$ to $R^3$ and $Q^1$ and $Q^2$, when they are alkyls, are $C_1$–$C_{24}$ and particularly $C_1$–$C_{18}$, and $C_5$–$C_{10}$ when they are cycloalkyls or aryls.

3. Process according to Claim 1 or 2, characterized in that the acidic catalyst is an ion exchanger, in particular a resin or a zeolite.

4. Process according to Claim 1 or 2, characterized in that the acidic catalyst is a silicoaluminate, in particular of the montmorillonite type.

5. Process according to Claim 1 or 2, characterized in that the acidic catalyst is an ion exchange resin with an aromatic polymeric structure or else with a perfluorinated aliphatic structure containing sulphonic groups and capable of being employed in bulk or fixed to an inorganic or organic support.

6. Process according to Claim 1 or 2, characterized in that the acidic catalyst is phosphoric acid impregnating a support.

7. Process according to one of Claims 1 to 6, characterized in that it is carried out at a temperature between room temperature and 200°C.

8. Process according to one of Claims 1 to 7, characterized in that it is operated at a pressure of 5 to 30 bars, more especially at 10 to 20 bars.

9. Process according to one of Claims 1 to 8, characterized in that the proportion of $H_2S$ employed is from 1 to 6 moles, and in particular 2 to 4 moles, per mole of thioether.

10. Process according to one of Claims 1 to 9, characterized in that the tertiary mercaptan formed, $(R^1R^2R^3)$–C–SH, is separated from the primary or secondary mercaptan, $Q^1Q^2$CH–SH, and employed for the production of a new quantity of thioether by reaction with an olefin or any other reactant capable of yielding the thioether, and the thioether is used anew for a preparation according to Claim 1.

11. Cyclic process according to Claim 10, characterized in that at least one of the groups $Q^1$ and $Q^2$ carries a functional group, especially –COOH, –CN, –OH, $-NH_2$, halogen, ether, ester, amide, nontertiary sulphide, sulphoxide or sulphone.

12. Process according to Claim 10, characterized in that the olefine is of the form

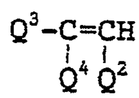 in which 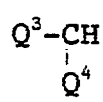

denotes $Q^1$ described above and/or $Q^3$ and $Q^4$ are hydrogens or hydrocarbon groups.